# EUROPEAN PATENT APPLICATION

(11) **EP 1 382 307 A1**
(43) Date of publication of application: **21.01.2004**
(21) Application number: 03015637.6
(22) Date of filing: 16.07.2003
(51) Int. Cl.: A61B 17/16

(54) **Medical handpiece and cutting tool therefore**

(30) Priority: 16.07.2002 JP 2002206351; 05.06.2003 JP 2003160649
(71) Applicant: Nakanishi Inc., Kanuma-shi, Tochigi 322-8666 (JP)
(72) Inventor: Nakanishi, Takasuke, c/o Nakanishi Inc., Kanuma-shi, Tochigi 322-8666 (JP)
(74) Representative: Wehnert, Werner, Dipl.-Ing.

(57) **Abstract**

A medical handpiece (10) is disclosed, which includes a cutting tool (1) having an elongate flexible shank (1e) and a burr (1a) provided at the distal end of the cutting tool (1), a tubular sheath (3) for receiving the cutting tool (1) therein and having an elongate tube portion (3c), an interposed member (4) interposed between the elongate tube portion (3c) and the shank (1e), and a handpiece body (5) connected to the sheath (3) and detachably holding the proximal end of the cutting tool (1) for transmitting driving force to the cutting tool (1). The elongate tube portion (3c) of the sheath (3) is malleable and deformable, and the shank (1e) of the cutting tool (1) and the interposed member (4) are deformable following malleable deformation of the elongate tube portion (3c). Also disclosed is a cutting tool (20) for use in the handpiece (10), which includes a shank (23) having a first section (23b) , and a second section (23a) having sufficient flexibility to be elastically deformed more easily than the first section (23b).

## Description

The present invention relates to a medical handpiece and a cutting tool therefor, in particular a medical handpiece having a cutting tool rotatably driven for cutting a treatment site, and the cutting tool.

Medical handpieces are commonly used. Some types of such handpieces have cutting tools, which are rotatably driven by driving sources, such as motors, to cut treatment site. Drills for transnasal. bone surgery are one of such handpieces, and used typically for cutting a bone in front of the pituitary gland of a patient by inserting the distal end of the drill through the patient's nasal cavity that has been expanded for the ease of insertion.

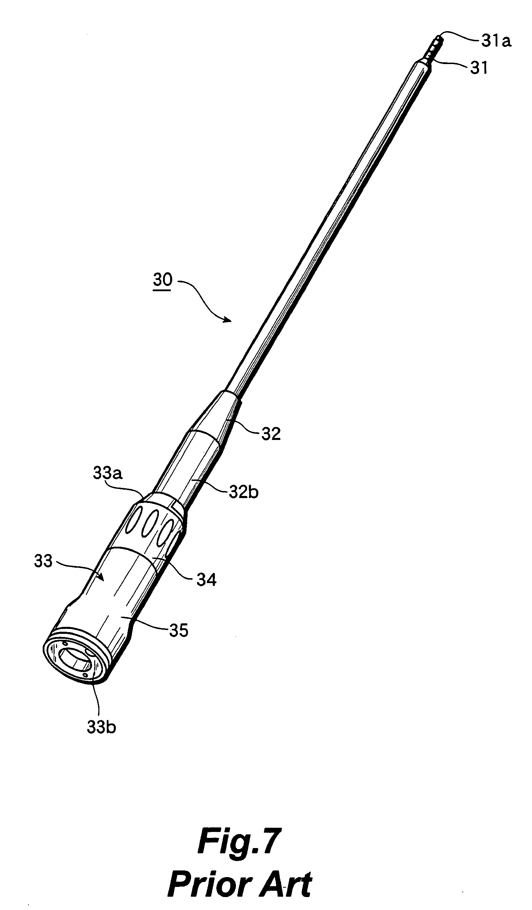

Fig. 7 shows an example of such conventional medical handpieces. Medical handpiece 30 is mainly composed of elongate cutting tool 31, tubular sheath 32, and handpiece body 33. The cutting tool 31, which has distal end 31a provided with a bit for cutting a treatment site, is inserted from its proximal end (not shown) into the sheath 32, and held in the handpiece body 33 on its proximal end. The handpiece body 33 has an internal chucking mechanism (not shown) for detachably holding the proximal end of the cutting tool 31. The chucking mechanism is operated by rotating twist ring 34. For example, upon clockwise rotation of the twist ring 34, the chuck mechanism is released to allow detachment of the cutting tool 31, whereas upon counterclockwise rotation of the twist ring 34, the chuck mechanism is closed to chuck the proximal end of the cutting tool 31. The sheath 32 has connector portion 32b, which is internally threaded to be screwed over the complementary threaded portion (not shown) on the distal end 33a of the handpiece body 33. The proximal end 33b of the handpiece body 33 is configured to be connected to a driving source (not shown) such as a motor. In use, an operator grips this medical handpiece 30 on grip portion 35, and rotatably drives the cutting tool 31 with the driving force from the driving source.

The sheath 32 of this handpiece 30 is straight and relatively firm, and the angle of the sheath 32 cannot be changed. Thus during the cutting operation, an operator has difficulties in getting a sight of the distal end 31a of the cutting tool 31, and in directing the distal end 31a to a desired site.

On the other hand, surgeons usually use a plurality of medical handpieces of varying sheath lengths for treatment of various sites, which accordingly requires a plurality of cutting tools of varying lengths. When the medical handpieces are to be cleaned or sterilized by autoclaving, the cutting tools are usually removed for separate cleaning, and reassembled into the rest of the handpiece after the cleaning. It is thus extremely complicated and bothersome for the surgeons, especially immediately before or during surgery, to match a cutting tool to a corresponding handpiece.

It is an object of the present invention to provide a medical handpiece which allows the operator to observe the tip of the cutting tool during surgery, and to adjust the sheath to an optimal angle for cutting.

It is another object of the present invention to provide a medical handpiece of which cutting tool is readily matched to the rest of the handpiece after being disassembled for cleaning or sterilizing.

It is yet another object of the present invention to provide a cutting tool for use in the medical handpiece which has an improved selective flexibility.

According to the present invention, there is provided a medical handpiece for cutting a treatment site, comprising:
a cutting tool having distal and proximal ends, and having an elongate flexible shank and a burr provided at said distal end of the cutting tool,
a generally tubular sheath for receiving the cutting tool therein, and having an elongate tube portion,
an interposed member interposed between the elongate tube portion of the sheath and the shank of the cutting tool,
a handpiece body connected to the sheath, and detachably holding said proximal end of the cutting tool for transmitting driving force from a drive source to the cutting tool,
wherein said elongate tube portion of the sheath is malleable and deformable, and said shank of the cutting tool and said interposed member are deformable following malleable deformation of the elongate tube portion.

According to one aspect of the present invention, the cutting tool may have a marker, and the sheath and/or the handpiece body may have a marker of the same color as that of the marker on the cutting tool. This facilitates matching of the cutting tool to the rest of the corresponding handpiece even when disassembled.

According to the present invention, there is also provided a cutting tool for use in the above-mentioned medical handpiece, comprising:
a burr at a distal end of the cutting tool for cutting a treatment site;
an elongate flexible shank extending proximally to the burr; and
a bearing contact portion positioned between the burr and the shank, and to be supported by a bearing in a medical handpiece when the cutting tool is mounted in the medical handpiece;
wherein said shank has a first section to be chucked in a handpiece body of the medical handpiece, and a second section having sufficient flexibility to be elastically deformed more easily than said first section.

The second section of the shank may be made with a smaller diameter than the diameter of the first section for giving the desired selective flexibility. With such a smaller diameter, the second section, and thus the entire shank may be made shorter, while maintaining sufficient flexibility to be deformed following the malleable deformation of the elongate tube portion of the sheath of the medical handpiece in which the cutting tool is mounted.

By way of example, preferred embodiments of the present invention will now be described with reference to the accompanying drawings, in which:
Fig. 1 is an exploded perspective view of an embodiment of the medical handpiece according to the present invention;
Fig. 2 is a longitudinal sectional view, partially elevational, of the distal part of the handpiece of Fig. 1, illustrating the cutting tool mounted in the sheath;
Fig. 3 is a longitudinal sectional view, partially elevational, similar to Fig. 2, illustrating the cutting tool partially drawn out of the sheath;
Fig. 4 is a schematic partial sectional view of the handpiece of Fig. 1;
Fig. 5 is a schematic perspective view of the handpiece of Fig. 1, illustrating deformation of the distal end portion of the handpiece;
Fig. 6 is a side view of another embodiment of the cutting tool according to the present invention; and
Fig. 7 is a schematic perspective view of a conventional medical handpiece.

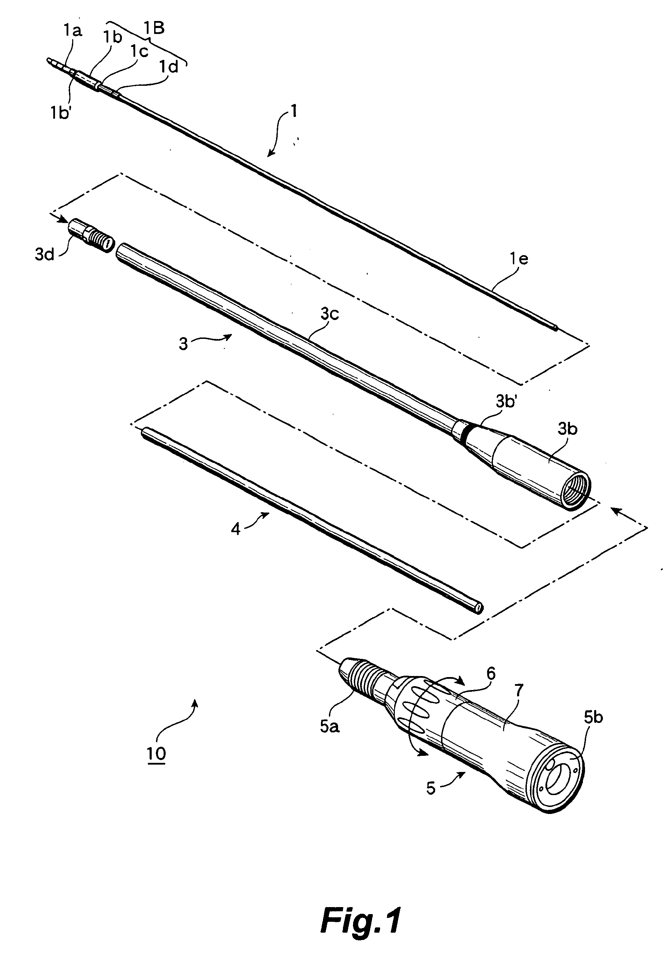

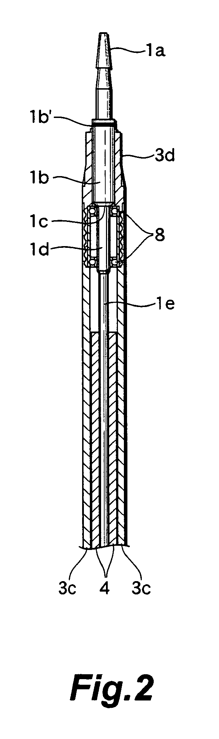

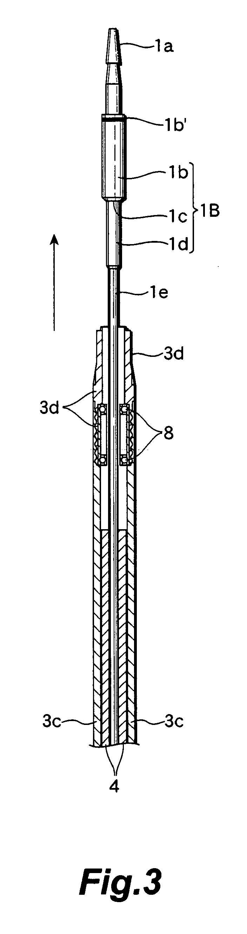

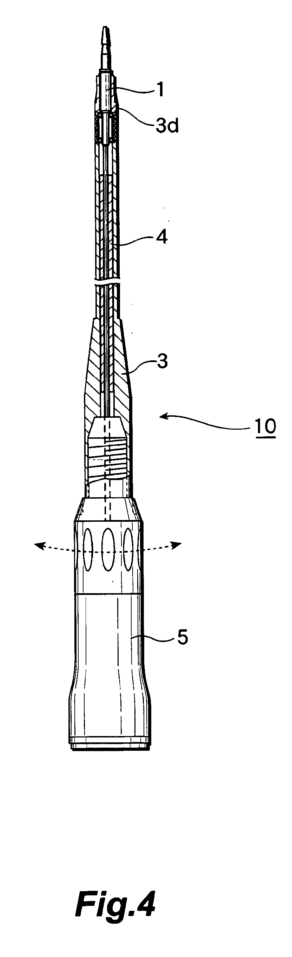

Referring to Figs. 1 to 5, medical handpiece 10 of the present invention includes cutting tool 1, sheath 3, interposed member 4, and handpiece body 5 as main components.

The cutting tool 1 has burr 1a at its distal end for cutting a treatment site, shank 1e extending proximal to the burr 1a, and bearing contact portion 1B positioned between the burr 1a and the shank 1e. These parts are separately formed and integrally connected by a suitable manner such as by welding or with an adhesive. The burr 1a may be formed, for example, by electrodeposition of diamond powders, attachment of a steel bit, or welding of a carbide bit. The shank 1e is elongate and flexible, and may be made, for example, of a metal such as stainless steel. The shank 1e is elastically deformable within its elastic limit, and may be deformed within about 30 degrees with respect to its longitudinal axis. The bearing contact portion 1B includes thickened part 1b and thinned part 1d, which form step 1c therebetween.

The sheath 3 is of generally tubular shape for receiving the cutting tool 1 therein, and has elongate tube portion 3c, connector portion 3b formed at the proximal end of the tube portion 3c, and cap 3d fixed to the distal end of the tube portion 3c.

The tube portion 3c is malleable and deformable, and may be malleably deformed within a predetermined range, for example, within 30 degrees with respect to its longitudinal axis, without breaking. The tube portion 3c may be made of a metal, such as stainless steel or titanium, and the wall thickness of the tube may be relatively thin, for example, about 0.1 to 0.8 mm. The tube portion 3c may be deformed or curved relatively easily when an operator applies force with his hand or fingers. Even by repeated deformation within the above range, the tube portion 3c will not break easily and stands for a relatively long time, though such deformation will not be made frequently. This is particularly true when the tube portion 3c is a metallic pipe having the wall thickness mentioned above.

The connector portion 3b has an enlarged diameter, and has internal threads for connection to the distal end 5a of the handpiece body 5 to be discussed later. The cap 3d is externally threaded, and screwed into the distal end of the tube portion 3c, which has corresponding internal threads. As shown in Figs . 2 and 3, the cap 3d has bearings 8 disposed therein for contacting and supporting the bearing contact portion 1B of the cutting tool 1. More specifically, the thinned part 1d of the bearing contact portion 1B is snugly received in the inner races of the bearings 8, with only the inner races of the bearings 8 abutting the step 1c. With this structure, the cutting tool 1 is positioned in place in the sheath 3, while the rotation of the cutting tool 1 is not disturbed.

Referring back to Fig. 1, according to one aspect of the present invention, the cutting tool 1 may have marker 1b at a location exposed outside when the cutting tool 1 is positioned in the sheath 3, for example, on the distal part of the thickened part 1b. On the other hand, the sheath 3 may have marker 3b' on its outer surface, for example, on the connector portion 3b. The markers 1b' and 3b' are of the same color, so that an operator may know at a glance which one of a plurality of cutting tools 1 corresponds to which one of a plurality of sheaths 3 without error. For this purpose, when a plurality of handpieces 10 are produced, cutting tools 1 of different lengths are marked with different colors, and sheaths of different lengths are also marked with different colors, with the corresponding length of the cutting tool and the sheath being marked with the same color. Thus even when a surgeon has a plurality of medical handpieces 10 of varying sheath lengths, and removes the cutting tools 1 for cleaning and sterilizing, the surgeon may easily match a cutting tool 1 to the rest of the corresponding handpiece 10. Alternatively, the marker 3b' may be provided on the outer surface of the handpiece body 5 to obtain the same advantage.

The interposed member 4 is tubular, and interposed between the tube portion 3c of the sheath 3 and the shank 1e of the cutting tool 1, i.e., the interposed member 4 is arranged inside the tube portion 3c, and the shank 1e is inserted into and received in the interposed member 4. The interposed member 4 acts as a sliding bearing for the shank 1e for supporting rotation of the cutting tool 1 around its longitudinal axis. For this purpose, the interposed member 4 preferably has sufficient heat resistance and wear resistance. On the other hand, the interposed member 4 should have sufficient flexibility to follow the malleable deformation of the outer sheath 3. In view of these, the interposed member 4 may be made of a suitable synthetic resin such as a fluororesin.

The handpiece body 5 has distal and proximal end portions 5a and 5b, grip 7, and twist ring 6 around a portion of the grip 7. The distal end portion 5a has external threads, which correspond to the internal threads of the connector portion 3b, for threaded connection between the sheath 3 and the handpiece body 5. The proximal end portion 5b is formed so as to be connected to a drive section such as a motor, for supply of power for rotationally driving the cutting tool 1. Though not shown in the drawings, the handpiece body 5 also has inside a chucking mechanism, which may be of a conventional structure. This chucking mechanism detachably holds the proximal end of the shank 1e of the cutting tool 1 inserted through the sheath 3 to place the cutting tool 1 in position for use. The chucking mechanism is structured to be operated by rotating the twist ring 6 with respect to the grip 7. For example, when the twist ring 6 is rotated clockwise with respect to the grip 7, the chucking mechanism is released to allow the cutting tool 1 to be detached. When the twist ring 6 is rotated counterclockwise, the chucking mechanism is closed to hold the shank 1e and the cutting tool 1 cannot be drawn out.

When the tube portion 3c of the sheath 3 is malleably deformed into a desired curved shape, the shank 1e of the cutting tool 1 and the interposed member 4 are deformed according to the deformation of the tube portion 3c, and the curved shape of the tube portion 3c is maintained for the surgery. In this state, when the cutting tool 1 is driven to rotate, the shank 1e rotates partly in contact with the interposed member 4, which acts as a sliding bearing for the shank 1e. Thus, even when the tube portion 3c of the sheath 3 is deformed, the cutting tool 1 may be rotated smoothly without contacting the inner surface of the sheath 3.

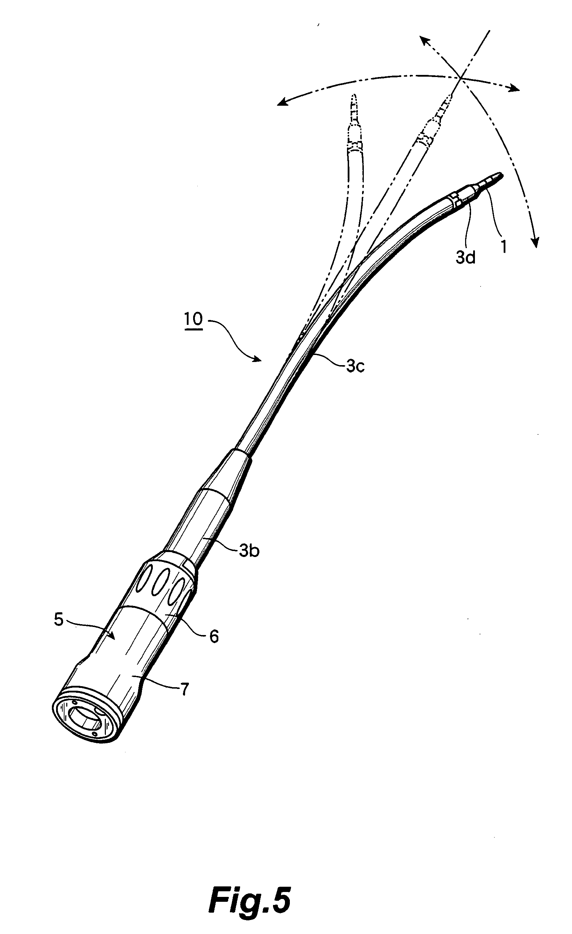

For use, the medical handpiece 10 of the above structure is connected at the proximal end portion 5b to a driving section such as a motor, with the tube portion 3c kept straight, or curved to a desired angle as shown by the arrows in Fig. 5. The operator grips the grip 7 of the handpiece 10, expands the nasal cavity of a patient, and inserts the distal portion of the handpiece 10 into the cavity. When the operator can see the tip of the cutting tool 1 with the current angle of the tube portion 3c, he rotatably drives the cutting tool 1 and cuts a treatment site. When the operator has difficulties in observing the tip of the tool 1, he can withdraw the handpiece 10 from the patient, malleably deform the tube portion 3c to properly adjust the curvature of the tube portion 3c, and re-insert the handpiece 10 into the nasal cavity for cutting the treatment site.

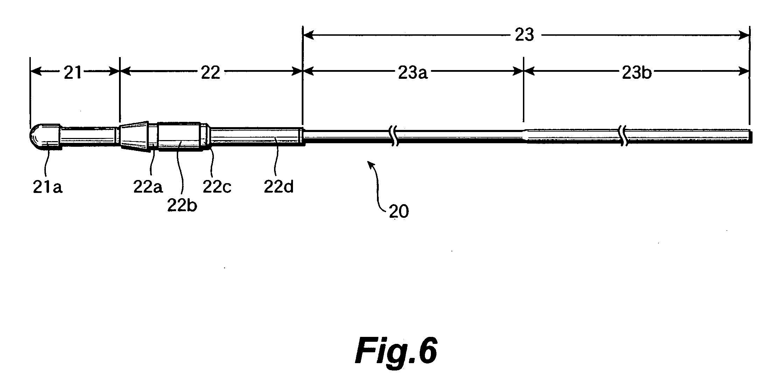

Fig. 6 shows another embodiment of the cutting tool of the present invention. Cutting tool 20 has cutting burr 21a and bearing contact portion 22, which are similar to the corresponding parts of the cutting tool 1 of Fig. 1, and shank 23. Distal end portion 21 including the cutting burr 21a and the shank 23 are integrally connected via the bearing contact portion 22, which has thickened part 22b, thinned part 22d, and step 22c formed therebetween. Marker 22a of a predetermined color is provided on the thickened part 22b.

The shank 23 is different from the corresponding part of the cutting tool 1, and has first section 23b and second section 23a located distal to the first section 23b. The first section 23b is to be held in the chucking mechanism in the handpiece body 5 when the cutting tool 1 is mounted for use. The second section 23a has a diameter smaller than that of the first section 23b. This smaller diameter allows the second section 23a to be elastically deformed more easily than the first section 23b. Preferred diameters of the first and second sections 23b and 23a may vary depending on the length of the shank 23, but, for example, with a shank 23 of about 100 mm long, the diameter of the first section 23b may be about 1 mm, and that of the second section 23a may be about 0.8 mm.

By providing such a relatively thinned second section 23a on the shank 23, even a relatively short cutting tool 20, which is to be used with a corresponding relatively short tube portion of a sheath, may be ensured to have sufficient selective flexibility for following the malleable deformation of the tube portion.

Though the flexibility of the second section 23a is enhanced compared to the first section 23b by relatively thinning the second section 23a in this embodiment, such flexibility may alternatively be enhanced by other suitable means or manners as will be appreciated by those skilled in the art.

The present invention has been discussed with reference to a drill for transnasal bone surgery as an example, but the present invention may also be applied to any medical handpieces having a cutting tool with a shank detachably inserted into an elongate tube portion of a sheath of the handpiece.

With the medical handpiece of the present invention, the tube portion of the sheath may be relatively easily deformed malleably into a desired curved shape simply by applying force with a hand or fingers. With such a desirably curved tube portion, an operator may give treatment using the handpiece while observing the tip of the cutting tool, so that surgery on a site that is hard to be cut with a conventional handpiece, may be facilitated.

With the markers of the same color provided on the cutting tool and the shank and/or the handpiece body, an operator may readily match the cutting tool to the rest of the corresponding handpiece, even after a plurality of handpieces are disassembled and cleaned together.

Further, with the cutting tool of the present invention, the selective flexibility of the shank is improved, so that, when the cutting tool is used with a medical handpiece of the present invention, the shank more smoothly follows the malleable deformation of the tube portion of the sheath in which the shank is disposed.

## Claims

1. A medical handpiece (10) for cutting a treatment site, comprising:
a cutting tool (1) having distal and proximal ends, and having an elongate flexible shank (1e) and a burr (1a) provided at said distal end of the cutting tool (1);
a generally tubular sheath (3) for receiving the cutting tool (1) therein, and having an elongate tube portion (3c);
an interposed member (4) interposed between the elongate tube portion (3c) of the sheath (3) and the shank (1e) of the cutting tool (1); and
a handpiece body (5) connected to the sheath (3) , and detachably holding said proximal end of the cutting tool (1) for transmitting driving force from a drive source to the cutting tool (1);
wherein said elongate tube portion (3c) of the sheath (3) is malleable and deformable, and said shank (1e) of the cutting tool (1) and said interposed member (4) are deformable following malleable deformation of the elongate tube portion (3c).

2. The medical handpiece (10) of claim 1, wherein said interposed member (4) is tubular and flexible.

3. The medical handpiece (10) of claim 1, wherein said interposed member (4) is made of a fluororesin, and acts as a sliding bearing for the shank (1e) of the cutting tool (1).

4. The medical handpiece of claim 1, wherein said cutting tool (1) has a marker (1b') and said sheath (3) and/or said handpiece body (5) has a marker (3b') of the same color as that of said marker (1b') on the cutting tool (1).

5. A cutting tool (20) for use in the medical handpiece (10) of claim 1, comprising:
a burr (21a) at a distal end (21) of the cutting tool (20) for cutting a treatment site;
an elongate flexible shank (23) extending proximally to the burr (21a); and
a bearing contact portion (22) positioned between the burr (21a) and the shank (23), and to be supported by a bearing in a medical handpiece when the cutting tool (20) is mounted in the medical handpiece;
wherein said shank (23) has a first section (23b) to be chucked in a handpiece body of the medical handpiece, and a second section (23a) having sufficient flexibility to be elastically deformed more easily than said first section (23b).

6. The cutting tool (20) of claim 5, wherein said second section (23a) has a diameter smaller than the diameter of the first section (23b).
